# EUROPEAN PATENT SPECIFICATION

(11) **EP 2 280 684 B1**
(45) Date of publication and mention of the grant of the patent: **04.12.2013**
(21) Application number: 09721645.1
(22) Date of filing: 06.03.2009
(51) Int. Cl.: A61P 17/00, A61K 35/407, A61K 8/27, A61K 8/64, A61K 8/67, A61Q 19/00, A61K 8/97, A61K 8/02, A61K 8/11, A61K 8/19, A61K 8/92

(54) **COMPOSITION FOR HAIR, SKIN AND NAIL HEALTH MAINTENANCE**
ZUSAMMENSETZUNG ZUR ERHALTUNG DER GESUNDHEIT VON HAAR, HAUT UND NÄGELN
COMPOSITION POUR LA SANTÉ DES CHEVEUX, DE LA PEAU ET DES ONGLES

(30) Priority: 18.03.2008 GB 0804957
(43) Date of publication of application: 09.02.2011
(73) Proprietor: Taylor, Robert Peter, Little Venice London W9 1AH (GB); Lalvani, Kartar Singh, London NW2 7HF (GB)
(72) Inventor: Taylor, Robert Peter, Little Venice London W9 1AH (GB); Lalvani, Kartar Singh, London NW2 7HF (GB)
(86) International application number: PCT/GB2009/000621
(87) International publication number: WO 2009/115769

(56) References cited:
- EP-A1- 1 514 554
- WO-A1-01/78674
- WO-A1-02/052955
- WO-A1-2006/000226
- WO-A1-2006/056293
- FR-A1- 2 737 849
- FR-A1- 2 889 057
- FR-A1- 2 894 776
- US-A- 5 514 672
- US-A1- 2006 251 750
- US-B2- 7 276 538
- BREDIF S. ET AL: "In vitro evaluation of an innovative nutraceutical compound for post-partum body remodeling" JOURNAL OF THE AMERICAN ACADEMY OF DERMATOLOGY, 1 February 2007 (2007-02-01), page AB90, XP005937028 C.V. MOSBY, ST. LOUIS, MO, US ISSN: 0190-9622
- ANONYMOUS: "SKIN, HAIR & NAILS FORMULA" INTERNET ARTICLE, [Online] 5 December 2007 (2007-12-05), XP002527473 Retrieved from the Internet: URL:http://www.feelgood-shop.com/Published Files/produktbeschreibungen/skin-hair-nail s-formula.htm> [retrieved on 2009-05-11]
- ANONYMOUS: "Was Haut, Haare und Nägel schöner macht" INTERNET ARTICLE, [Online] 9 December 2007 (2007-12-09), XP002527474 Retrieved from the Internet: URL:http://www.atlantis-pharm.com/haut%20h aare%20schoen.htm> [retrieved on 2009-05-11]

## Description

### TECHNICAL FIELD

This invention concerns a composition of biochemical or cosmeceutical constituents for use in the maintenance of the health of skin, hair and nails.

### BACKGROUND ART

We are living in an image conscious society where spending on beauty and beauty regimes has never been higher. In recent years this has extended into the supplement area as more people are aware of the benefits of seeking "beauty from within" and are increasingly adopting higher price "cosmeceuticals".

There is an increasing interest in not only maintaining the health of the hair and nails, but also a particular interest in maintaining the health of their skin.

Beauty supplement sales have grown by 5% in the last two years and this growth trend is expected to escalate with a market growth between 10% and 30% in the next 5 years. Therefore, beauty supplements can no longer be considered a niche market as more consumers become convinced of the benefits of seeking beauty through nutrition. Research showed that almost a third of the U.K. population, made up primarily of women and young girls, had used beauty supplements or would consider using them in the future. Most contain vitamins, antioxidants and omega-3 fatty acids.

Those in primary need are women between the ages of 35 and 45 years old who would like to protect against the visible signs of skin ageing. Also in primary need are women between 18 and 35 years old who are not yet concerned about the signs of ageing but who want to have healthy skin, hair, and nails in order to look good when socialising.

Those in secondary need are women of any age who have specific "problem" areas to address, most notably their skin, e.g. blemishes, redness, oily skin.

The supplements aimed at dealing with the health of the skin, hair and nails are remarkably diverse. There is no primary means, and many of the various means do not largely coincide with each other, or even coincide with each other at all. The following are the formulations of prominent supplements for skin, hair and nails :
Holland & Barrett - Skin Hair and Nails Formula : Vitamin A (from Beta Carotene), Vitamin E and Selenium.
Skin, Hair & Nails Tablets, By Natural Wealth : Cellulose (Plant Origin), Croscarmellose, Vegetable Stearic Acid, Silica, Vegetable Magnesium Stearate, Cellulose Coating, Mannitol.
Boots Skin Hair and Nails : Evening Primrose Oil, Gelatin, Calcium Pantothenate, Ascorbic Acid Glycerol, DL- Alpha- Tocopheryl Acetate, Magnesium Oxide, Zinc Sulphate, Ferrous Fumarate, Sodium Selenite, Pyridoxine Hydrochloride, Thiamine Nitrate, L-Cysteine Hydrochrloide, Silicon Dioxide, Beeswax, Riboflavin, Colour (Iron Oxide), Copper Sulphate, Manganese Sulphate, Folic Acid, Fractionated Coconut Oil, Biotin, Cholecalciferol, Antioxidant (DL-Alpha-Tocopherol.)
Fushi Skin, Hair & Nails Tonic : Burdock Root (Arctium Lappa), Artichoke Leaf (Cynara Scolymus), Horsetail (Equisetum Arvense), Sarsaparilla Root (Smilax Officinalis), Red Clover (Trifolium Pratense), Trikatu
La Style Skin, Hair And Nails : Thiamin (Vitamin B 1) 10mg, Riboflavin (Vitamin B2) 5mg, Niacin (Vitamin B3) 10mg, Pantothenic Acid (Vitamin B5) 40mg, Pyridoxine (Vitamin B6) 10mg, Folic Acid (Vitamin B9) 400mcg, Cobalamin (Vitamin B12) 10mcg, Iron 5mg, Chromium Picolinate 50mcg, Vitamin D3 5mcg, Vitamin C 60mg, Biotin 50mcg, Magnesium 75mg, Zinc 15mg, Iodine 150mcg, Manganese 1mg, Copper 1mg, Selenium 100mcg, Cystine 10mg, L-Lysine 50mg, Proline 50mg.
Vega Hair Skin Nails Formula : Amino Acid Complex (Soya Isolate) 100mg, Vitamin C (as Calcium Ascorbate) 50mg, RNA 50mg, Choline Bitartrate 40mg, Inositol 25mg, Magnesium (Oxide, Citrate and Amino Acid Chelate) 25mg, Calcium (from Citrate and Di Calcium Phosphate) 25mg, L-Methionine (Free Form) 15mg, Vitamin B3 (Nicotinamide) 10mg, Phosphorus (Di Calcium Phosphate) 10mg, Vitamin E (D-Alpha Tocopherol) 10iu 8.2mg, Vitamin B5 (as Calcium Pantothenate) 5mg, PABA 5mg, Vitamin B1 (Thiamine) 5mg, Vitamin B2 (Riboflavin) 5mg, Iron (Gluconate and Amino Acid Chelate) 5mg, Vitamin B6 (Pyridoxine) 2.5mg, Zinc (Citrate and Amino Acid Chelate) 2mg, Copper (Amino Acid Chelate) 25µg, Biotin 5µg, Iodine (as Potassium Iodide) 5µg, Vitamin B12 (Cyanocobalamin) 2.5µg

### DISCLOSURE OF INVENTION

This invention concerns a formulation aimed at supplementing the diet in order to provide constituents that will optimise the health of the hair, skin and nails. It has been developed for those individuals who while they are concerned about maintaining the health of their hair and nails have a particular interest in maintaining the health of their skin.

The inclusion of a nutri-dermal capsule provides essential Omega-3 and 6 fatty acids, which can help maintain a healthy looking complexion. It also includes a novel combination ingredients including lycopene; lutein and co-enzyme Q10 which help protect skin cells from the damaging effects of free radicals propagated by UV light and pollution.

The following is the formulation according to the present invention:
Daily dosages of the following constituents, provided in 1 tablet per day :
   Vitamin D3 - 5mcg
   Vitamin E (natural source) - 40 mg
   Vitamin C - 60mg
   Thiamin (vitamin B1) - 8 mg
   Riboflavin (vitamin B2) - 4 mg
   Niacin (vitamin B3) - 18 mg
   Vitamin B6 - 10 mg
   Folic Acid - 500 mcg
   Vitamin B12 - 9 mcg
   Biotin - 45 mcg
   Pantothenic acid - 40 mg
   Iron - 12 mg
   Magnesium - 75 mg
   Zinc - 15 mg
   Iodine - 200 mcg
   Manganese - 0.5mg
   Copper - 1 mg
   Chromium - 50 mcg
   Selenium - 100 mcg
   L-Cystine - 10 mg
   Natural Mixed Carotenoids - 2 mg
   Grape Seed Extract (95% proanthocyanidins) - 15mg
Plus, daily dosages of the following constituents, provided in 1 Nutri-dermal softgel capsule per day :
   Omega-3 Fish Oil - 300 mg
   Lutein - 6 mg
   Starflower Oil - 200 mg
   Blackcurrant Seed Oil - 50 mg
   Lycopene Extract 6% - 4 mg
   Co-Q10 - 5 mg
Starflower Oil, Omega-3 Fish Oil, and Blackcurrant Seed Oil together constitute more than 60% of the total weight of the composition.

In another version of the composition according to the present invention, Starflower Oil and Omega-3 Fish Oil constitute the majority of the composition, and are included with Proanthocyanidins, Coenzyme Q10, and Lycopene Extract 6%, and Blackcurrent Seed Oil.

### RDA / Active Levels :

The following are the recommended dietary allowances for some of the constituents, and the reasons for the level chosen :
Vitamin D3 (200iu) - 100% - Exact RDA level as a nutritional safeguard.
Natural Vitamin E -400% - Higher than RDA for increased antioxidant protection. Vitamin E is known to help the skin.
Vitamin C - 100% - Exact RDA level as a nutritional safeguard and important as a cofactor for the production of collagen.
Vitamin B1 - 571% - Because vitamin B1 is a water-soluble vitamin, any excess is excreted by the body making it safe to take at relatively high doses. Every cell of the body requires Vitamin B1.
Vitamin B2 - 250% - Because vitamin B2 is a water-soluble vitamin, the body excretes any excess. It is also of benefit to those who have cut red meat from their diet. A long-term intake of up to 200 mg daily is not considered harmful.
Vitamin B3 (Niacin) - 100% - Because Niacin is a water-soluble vitamin, the body excretes any excess. It is also of benefit to those who have cut red meat from their diet. A long-term intake of up to 500 mg daily is not considered harmful.
Vitamin B6 - 500% - Because vitamin B6 is a water-soluble vitamin, the body excretes any excess. A long-term intake of up to 200 mg daily is not considered harmful.
Folic Acid - 250% - To safeguard women who are pregnant or planning to become pregnant who are advised to take a daily supplement containing 400mcg of folic acid, to help prevent neural tube defects in the developing baby such as spina bifida. Folic acid is also involved in red blood cell production.
Vitamin B12 - 900% - Because vitamin B12 is a water-soluble vitamin, any excess is excreted by the body making it safe to take at relatively high doses. A long-term intake of up to 3000 mcg daily is not considered harmful.
Biotin - 30% - Widely distributed by many foods and also synthesised by the body.
Pantothenic Acid - 667% - Most people need to have Pantothenic Acid supplementation due to changing lifestyles. Primitive human diets provided greater amounts of this nutrient than is found in modem diets.
Iron - 86% - Excess iron in the tissues may lead to the production of free radicals and increase the need for vitamin E.
Magnesium - 25% - Below RDA level to take account of other dietary / supplement sources.
Zinc - 100% - RDA level.
Iodine - 133% - A lack of thyroxine hormone has a detrimental effect on the appearance of skin, hair and nails.
Manganese - No RDA level established
Copper - No RDA level established
Chromium GTF - No RDA level established
Selenium - No RDA level established
Cystine - No RDA level established
Natural Mixed Carotenoids - No RDA level established
Grape Seed Extract (95% proanthocyanidins) - No RDA level established
Omega-3 Fish Oil - No RDA level established
Lutein - No RDA level established
Starflower Oil - No RDA level established
Blackcurrant Seed Oil - No RDA level established
Lycopene Extract 6% - No RDA level established
Co-Q10 - No RDA level.

### Relevance of constituents

The composition provides a significant dietary level of nutrients and ensures that supplemental intake is within safety levels to help maintain healthy, skin, hair and nails. It provides particular support for skin. It also provides essential nutritional building blocks and protective factors for skin, hair and nails. The nutri-dermal capsule contains specialist ingredients including essential fatty acids for skin condition and additional antioxidants to help protect skin from free radical damage. One tablet will provide the ideal dietary level of nutrients and ensures that supplemental intake is within safety levels. One tablet a day will also ensure the skin, hair and nails receive all the nutrients they need from within. One nutri-dermal capsule provides a selection of nutrients specifically targeted at skin health.

Constituents are included for the following reasons :
Vitamin D3 plays a role in skin cell metabolism and growth and may be especially helpful for people with dry skin and psoriasis.
Vitamin E (natural source) scavenges free radicals, helping to protect against the ageing of skin tissues, which can cause wrinkles. It is also useful for stretch marks, scar tissue, and to reduce inflammation caused by sunburn.
Vitamin C is a central antioxidant and is also a cofactor in the production of collagen, which gives skin strength and elasticity. Hangnails and inflammation of the tissue surrounding the nail (paronychia) are linked to vitamin C deficiency.
The B complex vitamins : Thiamin (vitamin B1), Riboflavin (vitamin B2), Niacin (vitamin B3),Vitamin B6, Folic Acid,Vitamin B12, are involved in red blood cell production and blood circulation, thereby increasing the transport of nutrients to the dermis.
Biotin stimulates growth of body cells and related to hair growth.
Pantothenic Acid is essential in the metabolism of carbohydrate, fat & protein, for energy release.
Iron deficiency can produce "spoon" nails and/or vertical ridges and can cause nails to become thin.
Magnesium deficiency can lead to hair loss.
Zinc is needed for the formation of new nail material in the nail bed. Brittleness and white spots on nails are a common sign of zinc deficiency.
Iodine is essential for the production of thyroxine, a hormone which regulates metabolic rate. A lack of thyroxine can have a detrimental effect on the appearance of skin, hair and nails.
Manganese is vital for healthy skin because of its role in connective tissue metabolism.
Copper is necessary for hair strength and formation of hair follicles. Chromium plays a key role in fat and carbohydrate metabolism providing energy for skin cells. Plays a role in the body's glucose tolerance factor, important for individuals with a poor glucose balance which can in turn effect skin coloration and health.
Selenium is needed for antioxidants that help protect against skin damage caused by free radicals, UV light and pollution.
L-Cystine, the sulphur containing amino acid is necessary for skin and nail growth.
Natural Mixed Carotenoids helps protect skin from harmful effects of UV light.
Grape Seed Extract (95% proanthocyanidins) contains antioxidants to help protect cells from free radical damage and promote a healthy circulation.
Lutein helps protect skin from free radical damage particularly that induced by UV radiation.
Omega-3 Fish Oil helps cells repair and prevent damage. It also helps with the risk of skin cancer, help the body resist sunburn, and help reduce fine lines, wrinkles, and ease dry skin.
Blackcurrant Seed Oil provides the plant world's richest known natural source of Omega-3 and Omega-6. These important fatty acids may help to keep skin supple and toned.
Lycopene Extract 6% can play a valuable role in protecting vulnerable skin from the sun's UV radiation, which can contribute to premature skin damage.
Co-Q10 as well as being an antioxidant it has been shown to decrease the activity of certain enzymes that degrade collagen present in skin.

### BEST MODE FOR CARRYING OUT THE INVENTION

### Suggested Daily Intake/Dosage :

It is best to take 1 tablet and 1 capsule per day with a main meal, with a full glass of water or cold drink.

### Recommended Contra - Indications/Warnings :

The tablet and capsule should only be taken on a full stomach. This is recommended to ensure maximum absorption of nutrients and to avoid nausea which can occur when taking any tablets or capsules without food

No additional multivitamin is required.

### INDUSTRIAL APPLICABILITY

According to this invention, there is a composition for use in the maintenance of skin and nail and hair health. It consists of a daily intake of 1 tablet that consists of : 5mcg Vitamin D3, 40mg Vitamin E (natural source), 60mg Vitamin C, 8mg Thiamin (vitamin B1), 4mg Riboflavin (vitamin B2), 18mg Niacin (vitamin B3), 10mg Vitamin B6, 500mcg Folic Acid, 9mcg Vitamin B12, 45mcg Biotin, 40mg Pantothenic Acid, 12mg Iron, 75mg Magnesium, 15mg Zinc, 200mcg Iodine, 0.5mg Manganese, 1mg Copper, 50mcg Chromium, 100mcg Selenium, 10mg L-Cystine, 2mg Natural Mixed Carotenoids, 15mg Grape Seed Extract (95% proanthocyanidins); plus 1 Nutri-dermal softgel capsule that consists of : 300mg Omega-3 Fish Oil, 6mg Lutein, 200mg Starflower Oil, 50mg Blackcurrant Seed Oil, 4mg Lycopene Extract 6% , and 5mg Coenzyme Q10.

## Claims

1. A composition for use in hair, skin and nail health maintenance, consisting of a tablet containing : 5mcg Vitamin D3, 40mg Vitamin E (natural source), 60mg Vitamin C, 8mg Thiamin (vitamin B1), 4mg Riboflavin (vitamin B2), 18mg Niacin (vitamin B3), 10mg Vitamin B6, 500mcg Folic Acid, 9mcg Vitamin B12, 45mcg Biotin, 40mg Pantothenic Acid, 12mg Iron, 75mg Magnesium, 15mcg Zinc, 200mcg Iodine, 0.5mg Manganese, 1mg Copper, 50mcg Chromium, 100mcg Selenium, 10mg L-Cystine, 2mg Natural Mixed Carotenoids, and 15mg Grape Seed Extract (95% proanthocyanidins); plus a capsule containing : 300mg Omega-3 Fish Oil, 6mg Lutein, 200mg Starflower Oil, 50mg Blackcurrant Seed Oil, 4mg Lycopene Extract 6% , and 5mg Coenzyme Q10.

2. A composition for use in hair, skin and nail health maintenance, comprising Starflower oil and Omega-3 Fish Oil which constitute the majority of the composition, and Proanthocyanidins, Coenzyme Q10, Lycopene Extract 6%, and Blackcurrant Seed Oil.

## Patentansprüche

1. Zusammensetzung zur Verwendung in der Erhaltung der Gesundheit von Haar, Haut und Nägeln, bestehend aus einer Tablette, die Folgendes enthält: 5 µg Vitamin D3, 40 mg Vitamin E (aus natürlicher Quelle), 60 mg Vitamin C, 8 mg Thiamin (Vitamin B1), 4 mg Riboflavin (Vitamin B2), 18 mg Niacin (Vitamin B3), 10 mg Vitamin B6, 500 µg Folsäure, 9 µg Vitamin B12, 45 µg Biotin, 40 mg Pantothensäure, 12 mg Eisen, 75 mg Magnesium, 15 mg Zink, 200 µg Jod, 0,5 mg Mangan, 1 mg Kupfer, 50 µg Chrom, 100 µg Selen, 10 mg L-Cystin, 2 mg Mischung von natürlichen Carotinoiden und 15 mg Traubenkernextrakt (95% Proanthocyanidine) sowie einer Kapsel, die Folgendes enthält: 300 mg Omega-3-Fischöl, 6 mg Lutein, 200 mg Borretschöl, 50 mg Öl aus Schwarze-Johannisbeere-Kernen, 4 mg Lycopenextrakt 6%ig und 5 mg Koenzym Q10.

2. Zusammensetzung zur Verwendung in der Erhaltung der Gesundheit von Haar, Haut und Nägeln, die Borretschöl und Omega-3-Fischöl, welche den Großteil der Zusammensetzung ausmachen, und Proanthocyanidine, Koenzym Q10, Lycopenextrakt 6%ig und Öl aus Schwarze-Johannisbeere-Kernen umfasst.

## Revendications

1. Composition destinée à être utilisée dans le soin des cheveux, de la peau et des ongles, constituée d'un comprimé contenant : 5 mcg de vitamine D3, 40 mg de vitamine E (source naturelle), 60 mg de vitamine C, 8 mg de thiamine (vitamine B1), 4 mg de riboflavine (vitamine B2), 18 mg de niacine (vitamine B3), 10 mg de vitamine B6, 500 mcg d'acide folique, 9 mcg de vitamine B12, 45 mcg de biotine, 40 mg d'acide pantothénique, 12 mg de fer, 75 mg de magnésium, 15 mg de zinc, 200 mcg d'iode, 0,5 mg de manganèse, 1 mg de cuivre, 50 mcg de chrome, 100 mcg de sélénium, 10 mg de L-cystine, 2 mg de caroténoïdes mixtes naturels, et 15 mg d'extrait de pépin de raisin (95 % de proanthocyanidines) ; plus une capsule contenant : 300 mg d'oméga-3 huile de poisson, 6 mg de lutéine, 200 mg d'huile de bourrache, 50 mg d'huile de pépin de cassis, 4 mg d'extrait de lycopène à 6 %, et 5 mg de coenzyme Q10.

2. Composition destinée à être utilisée dans le soin des cheveux, de la peau et des ongles, comprenant de l'huile de bourrache et de l'oméga-3 huile de poisson qui constituent la majeure partie de la composition, et des proanthocyanidines, de la coenzyme Q10, d'extrait de lycopène à 6 %, et de l'huile de pépin de cassis.
